# EUROPEAN PATENT APPLICATION

(11) **EP 1 298 435 A2**
(43) Date of publication of application: **02.04.2003**
(21) Application number: 02021883.0
(22) Date of filing: 30.09.2002
(51) Int. Cl.: G01N 33/50, G01N 33/68

(54) **Assay for compounds affecting invertebrate cell secretory pathways**

(30) Priority: 28.09.2001 US 326134 P
(71) Applicant: Genoptera, LLC, South San Francisco, CA 94083-0511 (US); Bayer Corporation, Pittsburgh, PA 15205-9741 (US)
(72) Inventor: Ebens, Allen James, Jr., San Carlos, CA 94070 (US); Howard, Peter Kevin, Mill Valley, CA 94941 (US); Raming, Klaus, Redwood City, CA 94061 (US)
(74) Representative: Grünecker, Kinkeldey, Stockmair & Schwanhäusser Anwaltssozietät

(57) **Abstract**

The present invention provides assay methods for identifying agents that affect a secretory pathway in an invertebrate pest cell. The methods are useful for identifying agents that modulate a secretory pathway in an invertebrate pest cell, and that are therefore useful as pesticidal agents. The invention further provides methods for identifying secretory pathway proteins in an invertebrate pest cell, which secretory pathway proteins serve as targets for pesticides. The method further provides pesticidal agents identified using the assay methods of the instant invention.

## Description

### FIELD OF THE INVENTION

The invention relates to pesticide development, and to secretion of macromolecules in eukaryotic cells.

### BACKGROUND OF THE INVENTION

Export of macromolecules such as proteins from the cytosol of a eukaryotic cell is a complex process involving various membrane-bound organelles. Newly made secretory proteins are localized to the lumen of the rough endoplasmic reticulum (ER). Some proteins are secreted continuously (e.g., collagen secretion by fibroblasts; secretion of serum proteins from hepatocytes). Other proteins are not secreted continuously; instead, the secretion of these proteins is regulated (e.g., precursors of digestive enzymes, certain hormones, etc.). From the rough ER, secretory proteins migrate first to the interconnected vesicles known as the cis-Golgi reticulum on the cis face of the Golgi membrane complex. The proteins then undergo additional modifications as they migrate through the Golgi vesicles to the trans face and the trans-Golgi reticulum. Proteins move from one organelle to another in the secretory pathway by means of small transport vesicles. Plasma membrane proteins, e.g., glycoproteins, generally follow the same maturation pathway as continuously secreted proteins. Secretory and membrane proteins are folded, sorted, and transported to their ultimate destination. Secretory and membrane proteins may undergo various types of modifications, including formation of disulfide bonds, folding, addition and modification of carbohydrates, specific proteolytic cleavages, and formation of multichain proteins.

Many important macromolecules are exported from the cytosol, including neuropeptides that function as neurotransmitters and as hormones, secreted enzymes such as glycosidases and peptidases, enzymes that synthesize and modify macromolecules, such as chitin synthase and prolyl hydroxylase, and macromolecules such as fibronectin, laminin, collagen, chondroitin sulfate, and the like. For example, invertebrates use a wide range of secreted peptides as transmitters and hormones to regulate complex behavior, physiology and development. Within the peptidergic system secreted peptidases play a key role, being involved in both the biosynthesis of active peptide from the prohormone polypeptide and in peptide inactivation after release from a cell.

Proper function of the secretory apparatus is essential for the viability of cells and organisms. An example of a compound known to affect the above-described "conventional secretory pathway" is Brefeldin A, which collapses the Golgi apparatus. Brefeldin is a metabolite of the fungus *Eupenicillium brefeldianum*, which specifically and reversibly blocks protein transport from the endoplasmic reticulum (ER) to the Golgi apparatus in many cells types and species. These effects are generally accompanied by distinct morphological changes, including the apparent collapse of the Golgi stacks. The fast and reversible redistribution of intracellular membranes is accompanied by various specific and reversible effects on cellular protein traffic. These include blockage of the following processes: protein transport from the ER to the Golgi; protein secretion; vesicular assembly; antigen presentation; trans- and endocytosis; and viral assembly and budding.

The "conventional" secretory pathway (also referred to as the "classical secretory pathway) is generally common to eukaryotic cells. However, "alternative" or "non-conventional" secretory pathways have been described. For example, alternative secretory pathways have been described in yeast (Ziman et al. (1998) *Mol. Biol. Cell* 9:1565-1576) and plasmodium (Wiser et al. (1999) *Novartis Found Symp.* 226:199-211). Also, various mammalian secreted proteins are trafficked by non-conventional, Brefeldin A-resistant secretory mechanisms. Such proteins include FGF2 (Florkiewicz et al. (1995) J Cell Physiol. 3:388-399), Migration Inhibiting Factor (MIF) (O. Flieger et al. "A novel secretory pathway for the cytokine MIF", Abstract No. G.4, Joint Annual Meeting of Immunology of the German and Dutch Societies (DGfI, NVvI) November 29 - December 2, 2000, Duesseldorf, Germany), certain lectins (Sato et al. (1993) Exp Cell Res. 207(1):8-18), as well certain viral proteins (e.g. HSV; Miranda-Saksena et al.(2000) J Virol, 74(4): 1827-1839). In addition, secretory pathways in particular eukaryotic organisms may require one or more species-specific factors. See, e.g., Hwang et al. (2000) *J. Biol. Chem.* 275:17886-17893.

Pesticide development has traditionally focused on the chemical and physical properties of the pesticide itself, a relatively time-consuming and expensive process. As a consequence, efforts have been concentrated on the modification of pre-existing, well-validated compounds, rather than on the development of new pesticides. There is a need in the art for improved assays to detect compounds that are useful as pesticidal agents.

The present invention addresses this need by providing assays that detect agents that affect invertebrate cell secretory pathways. These assays identify targets for assay development, and enable target-based high-throughput screening which allows testing of an increased number of random chemical compounds to identify "hits", that is, chemical structures which cause inhibition in the assay. The described assays further enable pesticide product discovery by allowing rapid testing and chemical elaboration of "lead" structures based on the "hits" obtained from chemical library screening.

### Literature

Hwang et al. (2000) *J. Biol. Chem.* 275:17886-17893; Ziman et al. (1998) *Mol. Biol. Cell* 9:1565-1576; Sunio et al. (1999) *Mol. Biol. Cell* 10:847-859.

### SUMMARY OF THE INVENTION

The present invention provides assay methods for identifying agents that affect a secretory pathway in an invertebrate pest cell. The methods are useful for identifying agents that modulate a secretory pathway in an invertebrate pest cell, and that are therefore useful as pesticidal agents. In one embodiment of the invention, the method identifies agents that modulate a non-conventional secretory pathway. In another embodiment, the method identifies insect-specific inhibitors of macromolecule secretion. The invention further provides methods for identifying secretory pathway proteins in an invertebrate pest cell, which secretory pathway proteins serve as targets for pesticides. The method further provides pesticidal agents identified using the assay methods of the instant invention.

### FEATURES OF THE INVENTION

The present invention features n *in vitro* assay method for identifying an agent that modulates extracytosolic deposition of a macromolecule in an invertebrate cell. Of particular interest in many embodiments are agents that modulate extracytosolic deposition of a macromolecule in an insect pest cell. The methods generally involve contacting an invertebrate cell with a test agent, which invertebrate cell provides a detectable readout for distribution of the macromolecule in the cell; and determining the effect of the test agent on the distribution of the macromolecule in the cell. An effect on the distribution of the macromolecule, in comparison to a control invertebrate cell not contacted with the agent, indicates that the agent modulates extracytosolic deposition of the macromolecule in an invertebrate cell.

In some embodiments, the invertebrate cell is selected from the group consisting of Sf-9 cells, Sf-21 cells, S2 cells, Dmel, Kc, Hv1, Hv6, and Hi-5 cells. In other embodiments, the cell is present in an intact invertebrate organism.

In some embodiments, determining the effect of the test agent on the distribution of the macromlecule in the cell includes determining an altered subcellular distribution of the macromolecule compared to control cells. In some of these embodiments, the macromolecule is detected extracellularly. In other embodiments, the macromolecule is detected in the cytoplasm of the cell. In still other embodiments, the macromolecule is detected in a subcellular organelle.

In many embodiments of the invention, the macromolecule is a protein. In some of these embodiments, the altered subcellular distribution of the protein is detected by visual imaging of the protein. In some of these embodiments, the protein is detected by binding the protein to a labeled specific binding partner. In some embodiments, the specific binding partner is an antibody.

In some embodiments, the altered subcellular distribution is detected using a microscope and observing a single intracellular aggregrate of the protein compared to control cells that exhibit a diffuse, muliple-punctate intracellular distribution of the macromolecule. In some of these embodiments, the macromolecule is a protein, and prior to observing the cells using the microscope, the cells are fixed and contacted with an antibody specific to the protein. In these embodiments, the detectable read-out is provided by detecting antibody binding to the protein.

In other embodiments, the altered subcellular distribution is detected using automated image acquisition hardware.

In many embodiments, the invertebrate cell comprises a recombinant expression vector comprising a nucleotide sequence that encodes a fusion protein, which fusion protein includes a detectable marker protein fused in-frame to a secretion signal. In other embodiments, the invertebrate cell comprises a recombinant expression vector comprising a nucleotide sequence that encodes a fusion protein, which fusion protein comprises a detectable marker protein fused in-frame to a protein that normally enters a secretory pathway. In many of these embodiments, the detectable marker is selected from the group consisting of an immunological tag, a binding site, an enzyme that yields a detectable product, and a fluorescent protein.

In those embodiments in which the invertebrate cell comprises a first recombinant vector comprising a nucleotide sequence that encodes a first fusion protein comprising a signal peptide and a first marker protein, and a second recombinant vector comprising a nucleotide sequence that encodes a second marker protein, an altered subcellular distribution of the first marker protein, and a lack of an effect on levels of the second marker protein, indicates that the test agent specifically modulates a secretory pathway in the cell. In some of these embodiments, the first marker protein and the second detectable protein are each encoded by nucleotide sequences operably linked to an inducible promoter. In some embodiments, the inducible promoter is selected from the group consisting of a hormone-inducible promoter, a heat-inducible promoter, a metallothionein-inducible promoter, and a tetracycline-inducible promoter.

In some embodiments, the methods further include the step(s) of testing the agent in a counter screen using a mammalian cell line to determine whether the test agent affects extracytosolic deposition in a mammalian cell line.

The invention further features an agent identified using the method according to the invention. In some embodiments, the agent is a pesticidal agent.

The invention further features an *in vitro* assay method for identifying a modifier protein of an invertebrate secretory pathway. The method generally involves contacting an invertebrate cell that provides a detectable readout for distribution of a marker protein with an agent that specifically inhibits a putative modifier protein; and determining the effect of the agent on the distribution of the marker protein. An effect on the distribution of the marker protein, in comparison to a control cell not contacted with the agent, indicates that the inhibited protein is a modifier of an invertebrate secretory pathway.

In some embodiments, the agent is selected from the group consisting of a morpholino oligonucleotide, a double-stranded interfering RNA molecule, an antibody, an antisense nucleic acid molecule, and a dominant negative protein domain.

### BRIEF DESCRIPTIONS OF THE DRAWINGS

Figure 1 depicts an exemplary assay of the invention to detect agents that affect an invertebrate secretory pathway.
Figure 2 depicts further exemplary assays of the invention to detect agents that affect an invertebrate secretory pathway.

### DEFINITIONS

As used herein, the term "extracytosolic deposition" refers to the export of a macromolecule from the cytoplasm of a eukaryotic cell, or from the cytosol of a eukaryotic cell. Export of a macromolecule from the cytoplasm or cytosol of eukaryotic cell results in secretion of the macromolecule from the cell, association of the macromolecule with the cell surface, entry of the macromolecule into a membrane-limited organelle involved in export; or insertion of the macromolecule into the plasma membrane. Extracytosolic deposition encompasses movement of a macromolecule from the cytosol into a membrane-limited organelle involved in export, including Golgi vesicles, endoplasmic reticulum (including rough endoplasmic reticulum), endosomes, transport vesicles, secretory vesicles, Golgi apparatus, lysosomes, and the like.

As used herein, the term "macromolecule" refers to large molecules that are synthesized by a cell, including, but not limited to, proteins, glycoproteins, lipoproteins, glycolipids, and biological polymers such as chitin and collagen.

The terms "cargo," "marker protein," and "surrogate marker" are used interchangeably herein to refer to a macromolecule, such as a protein, that normally enters a conventional or alternative secretion pathway, and whose distribution in the cell serves to identify agents or targets that modulate the function of the secretory pathway. In the present invention, a macromolecule that enters a secretory pathway itself is detected, or a surrogate marker, such as a reporter molecule or other marker protein is detected. Thus, the term "macromolecule," in the context of a macromolecule that enters a secretory pathway, includes both the macromolecule that enters a secretory pathway, and a surrogate marker for the macromolecule.

The terms "secretory pathway" or "export pathway," are used interchangeably herein to refer to a pathway in a eukaryotic cell that results in or achieves extracytosolic deposition of a macromolecule.

The terms "classical" and "conventional," when used in the context of a secretory pathway, are used interchangeably herein to refer to a secretory pathway that involves movement of a protein from the rough endoplasmic reticulum to the Golgi apparatus. The terms "alternative" and "non-conventional," when used in the context of a secretory pathway, are used interchangeably herein to refer to a secretory pathway that does not involve movement of a protein from the rough endoplasmic reticulum to the Golgi apparatus.

Detection methods of the invention may be qualitative or quantitative. Thus, as used herein, the terms "detection," "determination," and the like, refer to both qualitative and quantitative determinations, and include "measuring."

As used herein, the term "pesticide" refers generally to chemicals, biological agents, and other compounds that adversely affect viability and/or fertility of an invertebrate pest, e.g., that kill, paralyze, sterilize, behaviorally incapacitate, or otherwise disable pest species in the areas of agricultural crop protection, human and animal health. As used herein, the term "invertebrate pest cell" refers to a cell of an invertebrate pest. Exemplary invertebrate pest species include parasites, disease vectors, and crop pest species, including insects, arachnids, nematodes, and the like. Invertebrate pest species also include those that are eradicated for aesthetic and hygienic purposes (*e*.*g*. ants, cockroaches, clothes moths, flour beetles, *etc.*)*,* home and garden applications, and protection of structures (including wood boring pests such as termites, and marine surface fouling organisms).

A "recombinant host cell", as used herein, denotes eukaryotic cells or cell lines cultured as unicellular entities which can be, or have been, used as recipients for recombinant vectors or other transfer polynucleotides, and include the progeny of the original cell into which the vector has been introduced. It is understood that the progeny of a single cell may not necessarily be completely identical in morphology or in genomic or total DNA complement as the original parent, due to natural, accidental, or deliberate mutation.

By "transformation" is meant a permanent or transient genetic change induced in a cell following incorporation of new nucleic acid molecules (i.e., nucleic acid molecules exogenous to the cell). Genetic change can be accomplished either by incorporation of the new nucleic acid molecule into the genome of the host cell, or by transient or stable maintenance of the new nucleic acid molecule as an episomal element. Where the cell is a eukaryotic cell, a permanent genetic change is generally achieved by introduction of the nucleic acid molecule into the genome of the cell. Such cells may be created and maintained in cell culture, created and maintained in recombinant non-human animals, or created in non-human recombinant animals and subsequently derived as cell lines and maintained in cell culture.

As used herein, a "recombinant" host cell is a host cell into which has been introduced (stably or transiently) an exogenous nucleic acid molecule, e.g., a recombinant vector, or construct, such as a construct encoding a fusion protein, as described herein. As used herein, the terms "native host cell" and "non-recombinant host cell," used interchangeably herein, refer to a cell that has not been genetically altered by introducing a recombinant vector into the host cell.

The terms "polynucleotide" and "nucleic acid molecule" are used interchangeably herein to refer to polymeric forms of nucleotides of any length. The polynucleotides may contain deoxyribonucleotides, ribonucleotides, and/or their analogs. Thus, the term includes DNA, RNA, double-stranded RNA, interfering RNA, and the like. Nucleotides may have any three-dimensional structure, and may perform any function, known or unknown. The term "polynucleotide" includes single-, double-stranded and triple helical molecules. Nucleic acids may be naturally occurring, *e*.*g*. DNA or RNA, or may be synthetic analogs, as known in the art.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "an agent" includes a plurality of such agents and reference to "the secretory pathway" includes reference to one or more such pathways and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION OF THE INVENTION

The present invention provides assay methods for identifying agents that affect a secretory pathway in an invertebrate pest cell. The methods are useful for identifying agents that inhibit a secretory pathway in an invertebrate pest cell, and that are therefore useful as pesticidal agents. The methods are also useful for identifying additional targets for pesticides, e.g., for identifying additional proteins that are involved in a secretory pathway in a cell of an invertebrate pest. The invention further provides nucleic acid molecules for use in the assay methods of the invention, recombinant vectors comprising the nucleic acid molecules, as well as recombinant host cells comprising the recombinant vectors. Recombinant vectors and host cells of the invention can be provided in the form of kits, for use in detecting agents that modulate a secretory pathway in an invertebrate pest cell. The invention further provides methods for isolating pesticidal agents. The invention further provides pesticidal agents identified using the assay methods of the instant invention.

### ASSAYS TO IDENTIFY AGENTS THAT AFFECT AN INVERTEBRATE PEST CELL SECRETORY PATHWAY

The present invention provides assay methods to identify agents that affect an invertebrate pest cell secretory pathway, e.g., that modulate extracytosolic deposition of a macromolecule, e.g., export of a macromolecule from the cytoplasm or the cytosol of an invertebrate pest cell. The methods generally involve contacting a cell with a test agent, which cell provides a detectable read-out for a secretory pathway; and determining the effect, if any, of the test agent on the detectable read-out. The macromolecule is referred to herein as a "cargo," i.e., a macromolecule such as a protein that normally enters the conventional or an alternative secretion pathway, and whose distribution (e.g., subcellular location) serves as a means to identify agents that affect an invertebrate pest cell secretory pathway, or (as described in more detail below) that serves as a means to identify additional components of a secretory pathway that are useful as targets for a pesticidal agent.

In some embodiments, the macromolecule (e.g., a cargo protein) itself is detected, e.g., using a labeled specific binding partner for the cargo protein. In other embodiments, a surrogate marker, such as a detectable marker protein is used, e.g., as part of a fusion protein that comprises a secretion signal and the detectable marker protein, or that comprises a cargo protein and the detectable marker protein. The cargo protein or the detectable marker protein can be detected at the cell surface, in the cytosol, or in a membrane-bound organelle.

The invention provides methods for identifying agents that affect a secretory pathway in an invertebrate pest cell, generally involve contacting a cell with a test agent, which cell provides a detectable read-out for a secretory pathway; and determining the effect, if any, of the test agent on the detectable read-out. A test agent of interest for further analysis is one that affects export of a macromolecule from the cytoplasm or the cytosol of the cell.

Assays typically include one or more controls that allow exclusion of agents that have a general effect on transcription and/or translation, or affects viability of the cell generally.

As used herein, the term "modulate" encompasses "increase" and "decrease." Agents that decrease (used interchangeably herein with "inhibit") an invertebrate pest cell secretory pathway are of particular interest in many embodiments.

In some embodiments, the invention provides assays that identify agents that modulate extracytosolic deposition of a macromolecule in an invertebrate pest cell, wherein the invertebrate pest cell is an insect cell. In these embodiments, the methods involve contacting an insect cell with a test agent, which insect cell provides a detectable readout for distribution of the macromolecule; and determining the effect of the test agent on the distribution of the macromolecule. An effect on the distribution of the macromolecule, in comparison to a control insect cell not contacted with the agent, indicates that the test agent modulates extracytosolic deposition of the macromolecule.

In some embodiments, the invention provides assays that identify agents that affect a non-conventional secretory pathway in an invertebrate pest cell. In some of these embodiments, the non-conventional secretory pathway is a secretory pathway that is insensitive to Brefeldin A, i.e., the pathway is affected by a test agent, but not by Brefeldin A. In other embodiments, the non-conventional secretory pathway is a secretory pathway that does not involve entry of the macromolecule into the Golgi. In some of these embodiments, the invertebrate pest cell is an insect cell.

In some embodiments, the methods involve contacting a cell that provides a detectable read-out with a test agent; and detecting an effect, if any, of the test agent on export of the cargo protein from the cytosol. In these embodiments, a cargo protein that is known to be exported from the cytosol or the cytoplasm of the cell is detected using any known methods, including, immunological methods (e.g., using an antibody specific for the cargo protein); assay methods involving contacting the macromolecule with a specific binding partner for the cargo protein; enzymatic assays, and the like. An effect on extracytosolic deposition of the cargo protein in the presence of the test agent indicates that the agent affects export from the cytoplasm or the cytosol.

In other embodiments, the cell comprises a recombinant vector that encodes a fusion protein comprising a cargo protein or an export signal, fused in-frame to a protein that is a detectable marker (the "fusion partner"). An effect on export of the fusion protein from the cytosol is determined by detecting the detectable marker in the cytoplasm (e.g., in the cytosol or in a membrane-bound organelle), in the medium, or on the cell surface. An effect on export of a fusion protein from the cytosol of the cell indicates that the agent is a candidate pesticidal compound.

The terms "agent," "substance," and "compound" are used interchangeably herein. Candidate agents encompass numerous chemical classes, typically synthetic, semi-synthetic, or naturally-occurring inorganic or organic molecules. Candidate agents may be small organic compounds having a molecular weight of more than 50 and less than about 2,500 daltons. Candidate agents may comprise functional groups necessary for structural interaction with proteins, particularly hydrogen bonding, and typically include at least an amine, carbonyl, hydroxyl or carboxyl group, and may contain at least two of the functional chemical groups. The candidate agents may comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Agents further encompass interfering RNA molecules, antibodies, and the like.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidation, etc. to produce structural analogs.

Where the screening assay is a binding assay, one or more of the molecules may be joined to a label, where the label can directly or indirectly provide a detectable signal. Various labels include radioisotopes, fluorescers, chemiluminescers, bioluminescers, enzymes, specific binding molecules, particles, e.g. magnetic particles, and the like. Specific binding molecules include pairs, such as biotin and streptavidin, digoxin and antidigoxin etc. For the specific binding members, the complementary member would normally be labeled with a molecule that provides for detection, in accordance with known procedures.

A variety of other reagents may be included in the screening assay. These include reagents like salts, neutral proteins, e.g. albumin, detergents, etc that are used to facilitate optimal protein-protein binding and/or reduce non-specific or background interactions. Reagents that improve the efficiency of the assay, such as protease inhibitors, nuclease inhibitors, anti-microbial agents, etc. may be used. The components are added in any order that provides for the requisite binding. Incubations are performed at any suitable temperature, typically between 4°C and 40°C. Incubation periods are selected for optimum activity, but may also be optimized to facilitate rapid high-throughput screening. Typically between 0.1 and 1 hour will be sufficient.

Agents of interest decrease extracytosolic deposition (e.g., decrease the extent or rate of extracytosolic deposition) of a macromolecule by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or more, when compared with a suitable control.

Agents that increase or decrease extracytosolic deposition of a macromolecule to the desired extent may be selected for further study, and assessed for cellular availability, cytotoxicity, biocompatibility, specificity for invertebrate pest cells, specificity for invertebrate pest organisms, absorption, distribution, metabolism, excretion, etc.

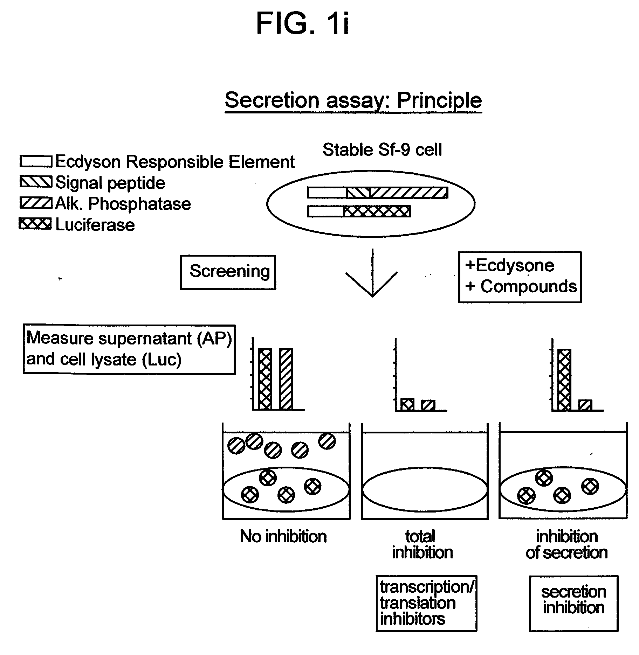

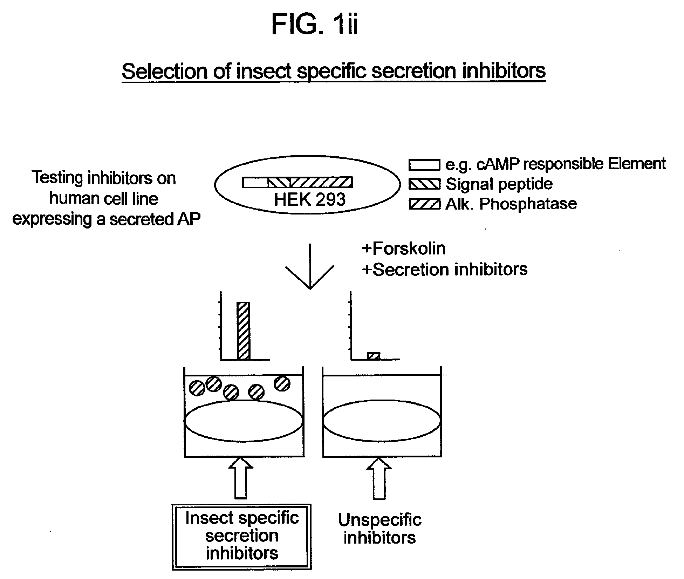

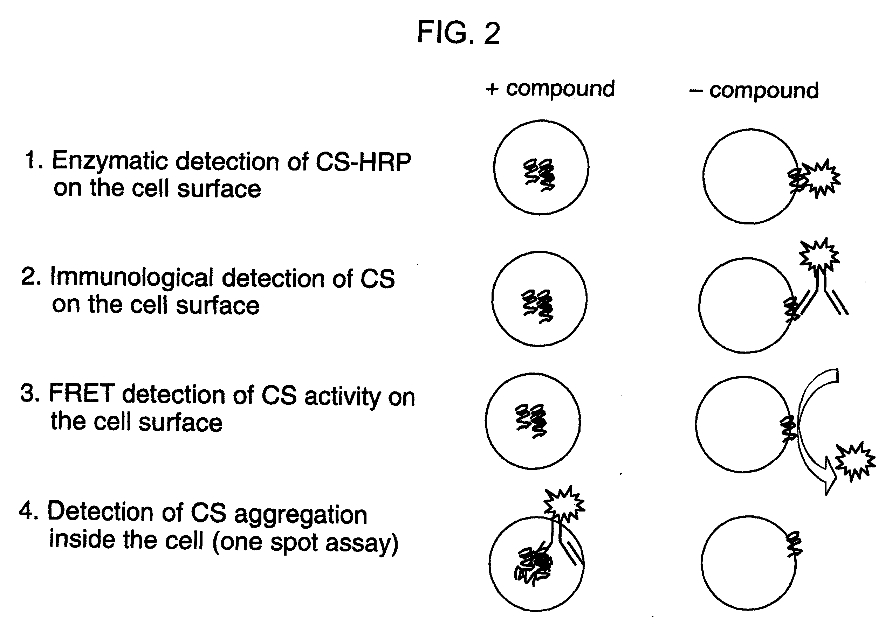

The subject assays can be designed in any of a variety of ways. Examples are shown in Figures 1 and 2. In some embodiments, the methods involve use of native, or non-recombinant cells. The cells are contacted with a test agent, and a macromolecule that is normally exported from the cytoplasm or the cytosol is detected. Detection of the macromolecule can be achieved by any known assay method. Immunological assay methods involve detection of the macromolecule using an antibody specific for the macromolecule, which antibody is detectably labeled either directly or indirectly (e.g., through use of a labeled secondary antibody). Enzymatic assays involve use of a substrate for the enzyme that gives rise to a detectable reaction product. Other methods involve use of a labeled specific binding partner for the macromolecule, including, but not limited to, a ligand for the macromolecule, and a regulatory protein that binds to the macromolecule.

In one particular embodiment of interest, the macromolecule is chitin synthase. Chitin synthase is normally found intracellularly in a multipunctate arrangement. When export of chitin synthase is disrupted, the enzyme is found in large aggregates inside the cell. The shift from a multipunctate arrangement to an aggregate arrangement is therefore an indication that export of chitin synthase from the cytosol is disrupted. Whether a shift from a multipunctate arrangement to an aggregate arrangement has occurred can be determined by examining the cells visually, e.g., under a microscope or other device suitable for *in vitro* visualization of cells (e.g., FMAT (Perkin-Elmer); or an automated image acquisition hardware, e.g. an Array Scan device (Cellomics); and the like).

In other embodiments, a recombinant cell is used, e.g., a recombinant insect cell, which recombinant cell comprises a recombinant vector comprising a nucleotide sequence that encodes a macromolecule that normally enters a secretory pathway; or that encodes a fusion protein comprising a secretion signal fused in-frame to another protein, such as a detectable marker; or that encodes a fusion protein comprising the cargo protein that includes a detectable marker protein, fused in-frame at the carboxyl terminus, the amino terminus, or internally to the cargo protein.

In some embodiments, a first recombinant expression construct is used that comprises a nucleic acid molecule comprising a nucleotide sequence that encodes a fusion protein that includes a secretion signal sequence (e.g., an insect cell-specific secretion signal sequence; or any known eukaryotic cell secretion signal sequence) fused in-frame to the amino or carboxyl terminus of a fusion partner, which fusion partner is a first detectable marker protein. The fusion protein-encoding nucleotide sequence is operably linked to a promoter for expression in the cells.

In some embodiments, the fusion protein-encoding nucleotide sequence is operably linked to an inducible promoter. The cells in *in vitro* cell culture are stably or transiently transfected with the first recombinant expression construct. The cells are contacted with a test agent, forming a test sample. Where the promoter is an inducible promoter, an inducer is added, resulting in transcription and translation of the fusion protein. If an inducer is added, the inducer is added simultaneously with, or shortly before or after the test agent (e.g., within about 1 hour of adding the test agent). A control sample has no test agent added. The culture supernatant is assayed for the presence of the first detectable marker. The effect of the agent on a secretory pathway is determined by comparing the subcellular distribution and/or location of first detectable marker in control and test samples.

To control for a general effect of the test agent on transcription and/or translation, cells are stably or transiently transfected with a second recombinant construct that includes a nucleotide sequence encoding a second selectable marker, the nucleotide sequence under transcriptional control of the same promoter as the first recombinant construct.

To determine whether the agent specifically affects an invertebrate pest cell secretory pathway, the first and second recombinant constructs are introduced into a non-insect cell line, and the assays performed on the non-insect cells. Non-limiting examples of non-insect cell lines that are suitable for use as control cells are HEK293 cells, COS cells, 3T3 cells, and the like. An agent that has an effect on secretion of the first detectable marker, but has no effect on transcription and translation of the second detectable marker, and further has no effect on macromolecule export in a non-insect cell, specifically affects an insect secretory pathway. Such agents are candidate pesticidal agents. However, as discussed in more detail below, agents that adversely affect a secretory pathway in both mammalian cells and insect cells may be candidate agents, if the absorption, distribution, metabolism, and excretion (ADME) is such that the agent adversely affects an invertebrate pest, but not a non-target organism (such as a plant or a mammal). Thus, to determine whether an agent is useful as a pesticide, the agent must be tested on whole organisms.

As one non-limiting example, a first recombinant expression construct for expression in insect cells is used, which contains a nucleotide sequence encoding a fusion protein that includes a secretion signal sequence fused in-frame to a fusion partner that serves as a detectable marker, e.g., an enzyme such as alkaline phosphatase. The nucleotide sequence is operably linked to an inducible promoter, e.g., an ecdysone response element, a heat-shock promoter, and the like. A second recombinant construct includes the ecdysone response element operably linked to a nucleotide sequence encoding luciferase. Insect cells are stably transfected with the first and second constructs. Ecdysone (or other appropriate inducer) is added to the cell culture, along with a test agent, forming a test sample. A control sample has no test agent added.

After a period of time, cell culture medium is assayed for the presence of alkaline phosphatase, and cell lysates are assayed for the presence of luciferase. Candidate agents of interest are those that do not affect the amount of luciferase produced, and that reduce the amount of alkaline phosphatase secreted into the medium.

In another embodiment, a first recombinant vector is introduced into an insect cell line, which first recombinant vector comprises a sequence that encodes a fusion protein that includes a detectable marker fused in-frame to the carboxyl terminus, the amino terminus, or at an internal site, of an insect protein that is normally exported from the cytoplasm and inserted into the membrane of an insect cell. The fusion protein-encoding nucleotide sequence is operably linked to a promoter for expression in insect cells. In some embodiments, the promoter is an inducible promoter. Insect cells in *in vitro* cell culture are stably or transiently transfected with the first recombinant expression construct.

The cells are contacted with a test agent, forming a test sample. Where the promoter is an inducible promoter, an inducer is added, resulting in transcription and translation of the fusion protein. If an inducer is added, the inducer is added simultaneously with, or shortly before or after the test agent (e.g., within about 1 hour of adding the test agent). A control sample has no test agent added. The fusion protein is detected in the cell membrane, and/or in the cytoplasm of the cell. The effect of the agent on a secretory pathway is determined by comparing the amount of first detectable marker in the cell membrane in control and test samples. Controls for a general effect on transcription and/or translation, and for insect-specificity, are included, as described above.

In general, the screening methods of the invention are *in vitro* assays, e.g., in cell culture using cell lines. However, assays can also be designed such that they are carried out in *in vivo* in multicellular organisms.

### Fusion proteins

Fusion proteins suitable for use in assay methods of the invention comprise an export pathway protein, or a secretion signal, fused in-frame to a protein that serves as a detectable marker (referred to herein as the "fusion partner"). Export pathway proteins include proteins that are secreted from a cell into the extracellular space, proteins that are exported from the cytoplasm and become associated with the plasma membrane following export from the cytoplasm, proteins that are inserted into the plasma membrane, and proteins that are themselves not exported from a cell, but which affect export of another protein from a cell.

### Export pathway proteins

Of particular interest in many embodiments are export pathway proteins that are found in invertebrate pest cells. Export pathway proteins ("cargo") are proteins that normally enter a conventional or alternative secretion pathway. Such export pathway proteins include, but are not limited to, proteins involved in chitin synthesis, such as chitin synthase; and any protein that is exported from the cytosol.

Proteins that are exported from an invertebrate pest cell include, but are not limited to, chitin synthase, insect prohormone convertase 2 (see, e.g., Hwang et al. (2000) *J. Biol. Chem.* 275:17886-17893); and the like.

The nucleotide and amino acid sequences of chitin synthase of various insects are publicly available. For example the following GenBank accession numbers provide insect chitin synthase nucleotide and amino acid sequences: AF229127, *Helicoverpa zea* chitin synthase; AF227729, *Drosophila melanogaster* chitin synthase; AF226626, *Chrysomya bezziana* chitin synthase; AF225705, *Haematobia irritans* chitin synthase; and AF221067, *Lucilia cuprina* chitin synthase. Any known insect chitin synthase, including the foregoing, can be used in an assay method of the invention.

Proteins that are exported from the cytoplasm and become associated with the plasma membrane include, but are not limited to, bride of sevenless (BOSS; GenBank Accession No. P22815), and the like.

Proteins that are themselves not exported from the cytoplasm but are involved in export of one or more other proteins from the cytoplasm include, but are not limited to, Sip1 (e.g. GenBank Accession No. AAF52282), signal recognition particle (Srp) receptor (e.g. GenBank Accession No. CAB01443, Srp54 (e.g. GenBank Accession No. AAF52825), Signal sequence receptor (SsR) (e.g. GenBank Accession No. AAA82458), synaptobrevin (e.g. GenBank Accession No. JC1522), syntaxin (e.g. GenBank Accession No. T13654), SNARE (e.g. GenBank Accession No. Q9VRL2), importin (e.g. GenBank Accession No. AAF34680), and the like.

### Secretion signals

Secretion signals that are suitable for use include any known eukaryotic secretion signal. Non-limiting examples include the secretion signal from insect prohormone convertase 2 (see, e.g., Hwang et al. (2000) *J. Biol. Chem.* 275:17886-17893.

Insect neuropeptides are described in the literature. See, e.g., Nassel et al. (1998) *Curr. Opin. Neurobiol*. 6:842-850. Insect secreted and membrane proteins are described in Goo et al. (1999) *Mol. Cells* 9:564-568. Insect prohormone convertase-2 is described in Siekhaus et al. (1999) *J. Neurosci.* 19:6942-6954; and Hwang et al. (2000) *J. Biol. Chem.* 275:17886-17893. The nucleotide and amino acid sequence of *Drosophila* prohormone convertase-2 (dPC2) is provided under GenBank Accession No. AF033117. Variants of dPC2 can also be used, as long as the variants promote extracytosolic deposition of the fusion protein.

### Fusion partners

Fusion partners that are suitable for use in the assay methods of the invention include any protein that can serve as a detectable marker, including, but not limited to, immunological tags; metal ion chelating peptides; chromogenic proteins; bioluminescent proteins; fluorescent proteins; enzymes that provide for a detectable product; proteins that are members of specific binding pairs; and the like.

Immunological tags are any polypeptide or antigenic epitope that is specifically bound by an antibody or epitope-binding fragment thereof, including, but not limited to, hemagglutinin; FLAG; c-myc; and the like.

Fluorescent proteins include, but are not limited to, a green fluorescent protein from *Aequoria victoria,* or fluorescent mutant thereof (e.g., cyan enhanced fluorescent protein, yellow enhanced fluorescent protein, and the like); a red fluorescent protein from Anthozoan species, or a fluorescent mutant thereof. See, e.g., U.S. Patent Nos. 6,066,476; 6,020,192; 5,985,577; 5,976,796; 5,968,750; 5,968,738; 5,958,713; 5,919,445 and 5,874,304; WO 01/27150; and Matz et al. (1999) *Nature Biotechnol.,* 17:969-973.

Enzymes that provide for a detectable product include, but are not limited to, β-galactosidase, horse radish peroxidase, luciferase, alkaline phosphatase, and the like.

Members of specific binding pairs that can be used include, but are not limited to, streptavidin (detected, e.g., using labeled biotin); avidin (e.g., streptavidin) (for binding to a biotinylated biomolecule); an antigen-binding polypeptide, e.g., an immunoglobulin (Ig) or epitope-binding fragment thereof (for binding to a biomolecule comprising an epitope recognized by the Ig); polynucleotide binding proteins (for binding to a polynucleotide), e.g., a transcription factor, a translation factor, and the like; Ni or Co chelate (to immobilize poly-histidine-tagged proteins); receptor-ligand systems, or other specific protein-protein interacting pairs; aptamers (e.g., nucleic acid ligands for three-dimensional molecular targets); lectins (for binding glycoproteins); lipids and phospholipids (binding to lipid-bindin proteins), e.g., phosphatidyl serine and annexin V.

### Recombinant vectors

The nucleotide sequence encoding the fusion protein can be inserted into any appropriate expression vector for the transcription and translation of the inserted protein-coding sequence. The expression vector will provide a transcriptional and translational initiation region, which may be inducible or constitutive, where the coding region is operably linked under the transcriptional control of the transcriptional initiation region, and a transcriptional and translational termination region. These control regions may be native to a gene encoding the export pathway protein, or may be derived from exogenous sources.

Various vectors are known in the art for introducing nucleic acid molecules into insect cells. A wide variety of nuclear polyhedrosis viruses have been isolated from insect species; many are in use as vectors for introducing nucleic acid molecules into insect cells. Various baculoviruses, including those that infect cotton bollworm, Helicoverpa zea, tobacco budworm, Heliothis virescens, Douglas fir tussock moth, Orgia pseudotsugata, gypsy moth, Lymantria dispar, alfalfa looper, Autographa californica, European pine fly, Neodiiprion sertifer, and codling moth, Laspeyresia pomonella, are suitable for use as vectors. Exemplary vectors include, but are not limited to, *Autographa califonica* multiple nucleopolyhedrovirus (AcMNPV) vectors, baculovirus vectors (pET11d, pLITMUS-29, and pFastBac1) *Orgyia pseudotsugata* multiple nucleopolyhedrovirus (OpMNPV), and the like. Many such vectors are commercially available.

To detect expression of a gene product, the expression vector can comprise a promoter (e.g., a polyhedrin promoter, and the like) operably linked to a subject nucleic acid molecule, one or more origins of replication, and, one or more selectable markers (e.g., hygromycin, etc.). Alternatively, recombinant expression vectors can be identified by assaying for the expression of a fusion protein based on the physical or functional properties of the fusion protein in *in vitro* assay systems (*e.g.* immunoassays).

Promoters can be constitutively active, or inducible. Constitutive promoters include an actin promoter, viral promoters that are constitutively active, etc. A wide variety of such promoters are known to those skilled in the art.

In many embodiments, the promoter is an inducible promoter. A wide variety of such promoters are known to those skilled in the art. Promoters that are inducible in insect cells include, but are not limited to, ecdysone-inducible promoters; promoters that are inducible with heat (e.g., promoters of heat-shock genes); metallothionein-inducible promoters; tetracycline-inducible promoters, and the like.

Ecdysone responsive elements are known in the art. Ecdysone response elements have been described in the *D. melanogaster* ecdysteroid-inducible polypeptide 40 (EIP40) (GenBank Accession No. AF095822); in the *D. melanogaster* larval serum protein 2 (GenBank Accession No. X97770) in which ecdysone response elements 5'-cggtcattggccg-3' and 5'-cgatcattgcagc-3' were identified; in the *D. melanogaster* gene for fat body protein 1 (GenBank Accession No. X69965); and in the *D. melanogaster* Broad-complex (GenBank Accession No. X62380).

Nucleic acid molecules comprising nucleotide sequences encoding a fusion protein may also be provided as part of a vector for propagating the nucleic acid molecules, which may then be excised from such vectors and inserted into an expression vector for introduction into an insect cell. A wide variety of such vectors are known in the art and need not be elaborated upon herein. Vectors include, but are not limited to, plasmids; cosmids; viral vectors; artificial chromosomes (YAC's, BAC's, etc.); mini-chromosomes; and the like. Vectors are amply described in numerous publications well known to those in the art, including, e.g., Short Protocols in Molecular Biology, (1999) F. Ausubel, et al., eds., Wiley & Sons.

### Host cells

Host cells suitable for use in assays of the present invention include isolated host cells, e.g., cells removed from an organism, cell lines, and the like, typically used in *in vitro* cell culture; and host cells that are part of an organism, e.g., whole invertebrate organisms, e.g. whole insects. Suitable host cells include recombinant host cells and native (non-recombinant) host cells.

In some embodiments, the methods use recombinant host cells, into which host cells a recombinant expression vector expressing the fusion protein or other surrogate marker is introduced. In many embodiments, host cells are insect cells. In general, such cells are capable of being culture *in vitro* over a period of time to allow synthesis of the fusion protein in the presence and absence of a test agent.

In other embodiments, the methods use non-recombinant host cells, e.g., a native insect cell (e.g., non-recombinant insect cell). In general, such cells are capable of being culture *in vitro* over a period of time.

Host cells suitable for use in an assay of the invention include any eukaryotic cell, including, but not limited to, mammalian cells and insect cells. In many embodiments, insect cells are of particular interest. Suitable insect cells include, but are not limited to, cells or cell lines isolated from *Heliothis virescens, Helicoverpa zea, Anticasia gemmatalis, Trichoplusia ni, Drosophila melanogaster, Spodoptera frugiperda, Spodoptera exigua, Bombyx mori,* and the like, and derivatives of any of the foregoing. Non-limiting examples of suitable cell lines are HIGH FIVE™, Sf9, *Drosophila S2* cells, expresSF™ cells, Dmel, Kc, Hv1, Hv6, Plodia cell lines, and the like.

In some embodiments, the host cell is present in a whole, intact organism. In some embodiments, the host cell that is in a whole organism is recombinant, e.g., comprises a construct that includes a nucleotide sequence encoding a macromolecule or surrogate marker (e.g. a fusion protein). In other embodiments, the host cell that is in a whole organism is a native, non-recombinant host cell. Non-limiting examples of such organisms are *Drosophila, Heliothis, Spodoptera*, and *C. elegans*.

To generate a recombinant host cell suitable for use in the methods of the invention, a recombinant vector comprising a nucleic acid molecule that encodes a fusion protein as described above is introduced into a host cell. Transformation of host cells may be accomplished in any convenient manner, where two representative means of transformation are treatment with divalent cation transformation compositions and electrotransformation. In transformation through divalent cation treatment, the host cells are typically incubated with the one or more divalent cations, e.g. CaCl₂, which serves to make the host cell permeable to the vector DNA. *See* Cohen et al. (1972) *Proc. Nat'l. Acad. Sci. USA* 69:2110. Other agents with which the host cells may also be incubated include DMSO, reducing agents, hexaminecobalt and the like, where such agents serve to improve the efficiency of transformation. In electrotransformation (also known as transformation by electroporation) host cells are subject to an electrical pulse in the presence of the vector in a manner sufficient for the vector to enter the host cells. *See* Dower et al. (1988) *Nucleic Acids Research* 16:6127.

Expression of heterologous genes in insects is accomplished as described in U.S. Patent No. 4,745,051; Friesen *et al*., "The Regulation of Baculovirus Gene Expression", in: *The Molecular Biology Of Baculoviruses* (1986) (W. Doerfler, ed.); EP 0 127,839; EP 0 155,476; and Vlak *et al., J. Gen. Virol.* (1988) *69:765-776;* Miller *et al., Ann. Rev. Microbiol.* (1988) *42*:177; Carbonell *et al., Gene* (1988) *73*:409; Maeda *et al., Nature* (1985) *315*:592-594; Lebacq-Verheyden *et al., Mol. Cell. Biol.* (1988) *8*:3129; Smith *et al., Proc. Natl. Acad. Sci. (USA)* (1985) *82*:8844; Miyajima *et al., Gene* (1987) *58:273;* and Martin *et al., DNA* (1988) *7*:99. Numerous baculoviral strains and variants and corresponding permissive insect host cells from hosts are described in Luckow *et al., Bio*/*Technology* (1988) *6*:47-55, Miller *et al., Generic Engineering* (1986) *8*:277-279, and Maeda *et al., Nature* (1985) *315*:592-594.

### Detection methods

Detection of the protein, whether it is an exported macromolecule ("cargo protein"), or the detectable marker portion of a fusion protein, or another surrogate marker, can be performed using any known assay. Assays include, but are not limited to, immunological assays, fluorescence resonance energy transfer (FRET)-based assay, bioluminescence resonance energy transfer (BRET)-based assays, enzymatic assays, visual assays, and the like. The specific assay used may depend in part on the nature of the protein as well as on the location of the protein. Thus, for example, a secreted protein can be detected in the culture medium, using an immunological assay, or an enzymatic assay, depending on the fusion partner. A protein that is exported from the cytosol and becomes associated with the plasma membrane such that the partner is accessible on the cell surface can be detected, e.g., using a fluorescence activated cell sorting assay, and the like.

### Visual assays

In some embodiments, determination of the effect of a test agent on export of a macromolecule from an invertebrate pest cell is a visual assay, e.g., the subcellular location of the fusion protein is determined visually using a microscope. Altered levels and/or distribution of a marker can be detected by automated image analysis using a device such as an FMAT device (Perkin-Elmer); an Array Scan device (Cellomics); and the like.

### FRET-based assays

Assay methods include FRET methods, in which the detectable marker protein is a fluorescent protein that serves as donor and/or acceptors in combination with a second fluorescent protein or dye, e.g., a fluorescent protein as described in Matz et al., (1999) *Nature Biotechnology* 17:969-973, a green fluorescent protein from *Aequoria victoria* or fluorescent mutant thereof, e.g., as described in U.S. Patent No. 6,066,476; 6,020,192; 5,985,577; 5,976,796; 5,968,750; 5,968,738; 5,958,713; 5,919,445; 5,874,304, the disclosures of which are herein incorporated by reference, other fluorescent dyes, e.g., coumarin and its derivatives, e.g. 7-amino-4-methylcoumarin, aminocoumarin, bodipy dyes, such as Bodipy FL, cascade blue, fluorescein and its derivatives, e.g. fluorescein isothiocyanate, Oregon green, rhodamine dyes, e.g. texas red, tetramethylrhodamine, eosins and erythrosins, cyanine dyes, e.g. Cy3 and Cy5, macrocyclic chelates of lanthanide ions, e.g. quantum dye, etc., chemilumescent dyes, e.g., luciferases, including those described in U.S. Patent Nos. 5,843,746; 5,700,673; 5,674,713; 5,618,722; 5,418,155; 5,330,906; 5,229,285; 5,221,623; 5,182,202; the disclosures of which are herein incorporated by reference.

### BRET-based assays

BRET-type assays may also be used. BRET is a protein-protein interaction assay based on energy transfer from a bioluminescent donor to a fluorescent acceptor protein. The BRET signal is measured by the amount of light emitted by the acceptor to the amount of light emitted by the donor. The ratio of these two values increases as the two proteins are brought into proximity. The BRET assay has been amply described in the literature. See, e.g., U.S. Patent Nos. 6,020,192; 5,968,750; and 5,874,304; and Xu et al. (1999) *Proc. Natl. Acad. Sci.* USA 96:151-156. BRET assays may be performed by genetically fusing a bioluminescent donor protein and a fluorescent acceptor protein independently to two different biological partners to make partner A-bioluminescent donor and partner B-fluorescent acceptor fusions. Changes in the interaction between the partner portions of the fusion proteins, modulated, e.g., by ligands or test compounds, can be monitored by a change in the ratio of light emitted by the bioluminescent and fluorescent portions of the fusion proteins. In this application, the proteins serve as donor and/or acceptor proteins.

### Immunological detection methods

Assay methods include immunological detection methods, wherein an antibody specific for a fusion partner is used to detect the presence of the fusion protein. Detection of specific binding of an antibody to a fusion protein, when compared to a suitable control, is an indication that fusion polypeptides are present in the sample. Suitable controls include a sample known not to contain the fusion polypeptide; and a sample contacted with an antibody not specific for the fusion partner, e.g., an anti-idiotype antibody. A variety of methods to detect specific antibody-antigen interactions are known in the art and can be used in the method, including, but not limited to, standard immunohistological methods, immunoprecipitation, an enzyme immunoassay (e.g., enzyme-linked immunoabsorbent assay, or ELISA), and a radioimmunoassay. In general, the fusion protein-specific antibody will be detectably labeled, either directly or indirectly.

Direct labels include radioisotopes; enzymes whose products are detectable (e.g., luciferase, β-galactosidase, and the like); fluorescent labels (e.g., fluorescein isothiocyanate, rhodamine, phycoerythrin, cyanin dyes, and the like); fluorescence emitting metals, e.g., ¹⁵²Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like. The antibody may be attached (coupled) to an insoluble support, such as a polystyrene plate or a bead. Indirect labels include second antibodies specific for fusion protein-specific antibodies, wherein the second antibody is labeled as described above; and members of specific binding pairs, e.g., biotin-avidin, and the like. The biological sample may be brought into contact with an immobilized on a solid support or carrier, such as nitrocellulose, that is capable of immobilizing cells, cell particles, or soluble proteins. The support may then be washed with suitable buffers, followed by contacting with a detectably-labeled fusion protein-specific antibody. Detection methods are known in the art and will be chosen as appropriate to the signal emitted by the detectable label. Detection is generally accomplished in comparison to suitable controls, and to appropriate standards.

### FACS-based assays

Fluorescence activated cell sorting (FACS)-based methods are useful for detecting fusion proteins that are exported from the cytoplasm and become associated with the cell membrane such that the fusion partner is accessible on the cell surface. FACS methods are well known in the art, and have been amply described in the literature. For example, FACS methods are described in U.S. Patent Nos. 5,968,738 and 5,804,387; the disclosures of which are herein incorporated by reference.

### SCREENING ASSAYS TO IDENTIFY EXPORT PATHWAY PROTEINS

The present invention further provides assay methods to identify additional targets for pesticidal agents, which targets are involved in extracytosolic deposition of macromolecules. Proteins involved in extracytosolic deposition of macromolecules are referred to herein as export pathway proteins. The methods generally involve contacting a cell that provides a detectable read-out with an agent that affects the activity or expression of a protein involved in extracytosolic deposition of a macromolecule; and identifying the protein affected.

Agents that affect the activity or expression of a protein involved in extracytosolic deposition of a macromolecule include, but are not limited to, interfering RNA (RNAi) molecules; antisense nucleic acid molecules, transposable DNA elements, chemical libraries; morpholino antisense oligonucleotides; antibodies, including antigen-binding fragments; dominant negative protein domains; and the like, which inactivate or disrupt a protein involved in an invertebrate pest cell secretory pathway.

### Morpholino antisense oligonucleotides

Morpholino antisense oligonucleotides and preparation of same are amply described in the literature. See, e.g., Summerton and Weller (1997) *Antisense Nucl. Acid Drug Dev.* 7:187-195; Summerton (1999) *Biochim. Biophys. Acta* 1489:141-158; Ghosh and Iversen (2000) *Antisense Nucl. Acid Drug Dev.* 10:263-274; Ekker (2000) *Yeast* 17:302-306.

### Dominant negative protein domains

A "dominant negative protein domain" refers to a protein domain that, when produced in a host cell, disrupts the function of the corresponding wild-type (e.g., full-length) protein the host cell. Such dominant negative protein domains can act, inter alia, by providing to the cell single or multiple copies of a portion of the full-length protein, which portions bind a natural ligand (e.g., a substrate, a counter-receptor, a modulator protein, any protein with which the protein normally interacts, and the like) of the full-length protein, or which bind to a subunit (where the protein normally exists as a multimer) that the full-length protein normally binds to, thereby disrupting the function of the full-length protein. For example, in the presence of an association domain, a full-length protein can form inactive multimeric complexes.

### Antibodies

Antibodies of interest include antibodies that bind to epitopes on proteins that are known to be involved in a secretory pathway, which epitopes may be shared by other proteins of unknown identity but with similar function. Antibodies to suitable epitopes on protein surfaces may decrease the abundance, and thereby indirectly decrease the activity, of the wild-type active form of a target protein by aggregating active forms into complexes with less or minimal activity as compared to the wild-type unaggregated wild-type form. Alternatively, antibodies may directly decrease protein activity by, e.g., interacting directly with active sites or by blocking access of substrates to active sites.

Antibodies can be introduced into cells in numerous fashions, including, for example, microinjection of antibodies into a cell (Morgan et al., 1988, Immunology Today 9:84-86) or transforming hybridoma mRNA encoding a desired antibody into a cell (Burke et al., 1984, Cell 36:847-858). In a further technique, recombinant antibodies can be engineering and expressed in a wide variety of non-lymphoid cell types to bind to target proteins as well as to block target protein activities (Biocca et al, 1995, Trends in Cell Biology 5:248-252). Sequences encoding the variable regions of an antibody are cloned into various engineered antibody formats, including, for example, whole antibody, Fab fragments, Fv fragments, single chain Fv fragments (V_{H} and V_{L} regions united by a peptide linker) ("ScFv" fragments), diabodies (two associated ScFv fragments with different specificities), and so forth (Hayden et al., 1997, Current opinion in Immunology 9:210-212).

Intracellularly expressed antibodies of the various formats can be targeted into cellular compartments (e.g., the cytoplasm, the nucleus, the mitochondria, etc.) by expressing them as fusions with the various known intracellular trafficking sequences (Bradbury et al., 1995, Antibody Engineering (vol. 2) (Borrebaeck ed.), pp 295-361, IRL Press). In particular, the ScFv format appears to be particularly suitable for cytoplasmic targeting.

For preparation of monoclonal antibodies directed towards a target protein, any technique that provides for the production of antibody molecules by continuous cell lines in culture may be used. Such techniques include, but are not limited to, the hybridoma technique originally developed by Kohler and Milstein (1975, Nature 256: 495-497), the trioma technique, the human B-cell hybridoma technique (Kozbor et al., 1983, Immunology Today 4: 72), and the EBV hybridoma technique to produce human monoclonal antibodies (Cole et al., 1985, in Monoclonal Antibodies and Cancer Therapy, Alan R. Liss, Inc., pp. 77-96). In an additional embodiment of the invention, monoclonal antibodies can be produced in germ-free animals utilizing recent technology (PCT/US90/02545).

Additionally, where monoclonal antibodies are advantageous, they can be alternatively selected from large antibody libraries using the techniques of phage display (Marks et al., 1992, J. Biol. Chem. 267:16007-16010). Using this technique, libraries of up to 10¹² antibodies have been expressed on the surface of fd filamentous phage, creating a "single pot" in vitro immune system of antibodies available for the selection of monoclonal antibodies (Griffiths et al., 1994, EMBO J. 13:3245-3260). Selection of antibodies from such libraries can be done by techniques known in the art, including contacting the phage to immobilized target protein, selecting and cloning phage bound to the target, and subcloning the sequences encoding the antibody variable regions into an appropriate vector expressing a desired antibody format.

Techniques described for the production of single chain antibodies (U.S. Pat. No. 4,946,778) can be adapted to produce single chain antibodies specific to the target protein. Fab expression libraries (Huse et al., 1989, Science 246: 1275-1281) can also be used.

### Antisense nucleic acid molecules

Antisense nucleic acids are also of interest as agents that modulate an export pathway. An "antisense" nucleic acid as used herein refers to a nucleic acid capable of hybridizing to a sequence-specific (e.g., non-poly A) portion of the target RNA, for example its translation initiation region, by virtue of some sequence complementarity to a coding and/or non-coding region. The antisense nucleic acid can be an oligonucleotide that is double-stranded or single-stranded, RNA or DNA or a modification or derivative thereof, which can be directly administered to a cell or which can be produced intracellularly by transcription of exogenous, introduced sequences in quantities sufficient to inhibit translation of the target RNA. Antisense polynucleotides, and methods of using such, are described in numerous publications, including, e.g., "Antisense Technology: A Practical Approach" Lichtenstein and Nellen, eds. (1997) IRL Press.

The antisense nucleic acid molecules can be DNA or RNA or chimeric mixtures or derivatives or modified versions thereof, single-stranded or double-stranded. The antisense nucleic acid molecules can be modified at the base moiety, sugar moiety, or phosphate backbone. The antisense nucleic acid molecules may include other appending groups such as peptides, or agents facilitating transport across the cell membrane (see, e.g., Letsinger et al., 1989, Proc. Natl. Acad. Sci. U.S.A. 86: 6553-6556; Lemaitre et al., 1987, Proc. Natl. Acad. Sci. 84: 648-652; PCT Publication No. WO 88/09810), hybridization-triggered cleavage agents (see, e.g., Krol et al., 1988, BioTechniques 6: 958-976) or intercalating agents (see, e.g., Zon, 1988, Pharm. Res. 5: 539-549).

Antisense oligonucleotides will generally be at least about 7, usually at least about 12, more usually at least about 20 nucleotides in length, and not more than about 500, usually not more than about 50, more usually not more than about 35 nucleotides in length, where the length is governed by efficiency of inhibition, specificity, including absence of cross-reactivity, and the like. It has been found that short oligonucleotides, of from 7 to 8 bases in length, can be strong and selective inhibitors of gene expression (see Wagner et al. (1996) *Nature Biotechnology* 14:840-844).

The antisense nucleic acid molecule may be conjugated to another molecule, e.g., a peptide, hybridization triggered cross-linking agent, transport agent, hybridization-triggered cleavage agent, etc.

Antisense nucleic acid molecules are synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16: 3209), methylphosphonate oligonucleotides can be prepared by use of controlled pore glass polymer supports (Sarin et al., 1988, Proc. Natl. Acad. Sci. U.S.A. 85: 7448-7451), etc. In another embodiment, the oligonucleotide is a 2'-O-methylribonucleotide (Inoue et al., 1987, Nucl. Acids Res. 15: 6131-6148), or a chimeric RNA-DNA analog (Inoue et al., 1987, FEBS Lett. 215: 327-330).

In an alternative embodiment, the antisense nucleic acids of the invention are produced intracellularly by transcription from an exogenous sequence. For example, a vector can be introduced into a cell, within which cell the vector or a portion thereof is transcribed, producing an antisense nucleic acid (RNA) of the invention. Such a vector would contain a sequence encoding the antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells.

The antisense nucleic acids comprise a sequence complementary to at least a portion of a target RNA species. However, absolute complementarity, although preferred, is not required. A sequence "complementary to at least a portion of an RNA," as referred to herein, means a sequence having sufficient complementarity to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a target RNA it may contain and still form a stable duplex (or triplex, as the case may be). One skilled in the art can ascertain a tolerable degree of mismatch by use of standard procedures to determine the melting point of the hybridized complex. The amount of antisense nucleic acid that will be effective in the inhibiting translation of the target RNA can be determined by standard assay techniques.

Therefore, antisense nucleic acids can be routinely designed to target virtually any mRNA sequence, and a cell can be routinely transformed with or exposed to nucleic acids coding for such antisense sequences such that an effective amount of the antisense nucleic acid is expressed. Accordingly the translation of virtually any RNA species in a cell can be inhibited.

### Chemical libraries

Preparation and screening of combinatorial chemical libraries is well known to those of skill in the art. Such combinatorial chemical libraries include, but are not limited to, peptide libraries (see, e.g., U.S. Pat. No. 5,010,175, Furka, Int. J. Pept. Prot. Res. 37:487-493 (1991) and Houghton et al., Nature 354:84-88 (1991)). Other chemistries for generating chemical diversity libraries can also be used. Such chemistries include, but are not limited to: peptoids (PCT Publication No. WO 91/19735), encoded peptides (PCT Publication WO 93/20242), random bio-oligomers (PCT Publication No. WO 92/00091), benzodiazepines (U.S. Pat. No. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J. Amer. Chem. Soc. 114:6568 (1992)), nonpeptidal peptidomimetics with β-D-glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho et al., Science 261:1303 (1993)), and/or peptidyl phosphonates (Campbell et al., J. Org. Chem. 59:658 (1994)), nucleic acid libraries, peptide nucleic acid libraries (see, e.g., U.S. Pat. No. 5,539,083), antibody libraries (see, e.g., Vaughn et al., Nature Biotechnology, 14(3):309-314 (1996)), carbohydrate libraries (see, e.g., Liang et al. *Science* 274:1520-1522 (1996) and U.S. Pat. No. 5,593,853), small organic molecule libraries; isoprenoids, U.S. Pat. No. 5,569,588; thiazolidinones and metathiazanones, U.S. Pat. No. 5,549,974; pyrrolidines, U.S. Pat. Nos. 5,525,735 and 5,519,134; morpholino compounds, U.S. Pat. No. 5,506,337; benzodiazepines, 5,288,514, and the like).

### Interfering RNA

In many embodiments the agent that affects the activity or expression of a protein involved in extracytosolic deposition of a macromolecule is an interfering RNAi molecule. RNAi fragments, e.g., double-stranded (ds) RNAi, can be used to generate loss-of-function phenotypes. Another method for generating loss-of-function phenotypes is by double-stranded RNA interference (dsRNAi). This method is based on the interfering properties of double-stranded RNA derived from the coding regions of gene, and has proven to be of great utility in genetic studies of *C. elegans* (Fire *et al*., Nature (1998) 391:806-811), and can also be used to generate loss-of-function phenotypes in *Drosophila* (Kennerdell and Carthew, Cell (1998) 95:1017-1026; Misquitta and Patterson PNAS (1999) 96:1451-1456). In one example of this method, complementary sense and antisense RNAs derived from a substantial portion of a gene of interest, such as an invertebrate receptor gene, are synthesized *in vitro.* The resulting sense and antisense RNAs are annealed in an injection buffer, and the double-stranded RNA injected or otherwise introduced into animals or cells in culture (such as in their food or by soaking in the buffer containing the RNA). Progeny of the injected animals are then inspected for phenotypes of interest (PCT publication no. WO99/32619). See also, Clemens et al. (2000) Proc. Natl. Acad. Sci. USA 97:6499-6503.

In some embodiments, a library of RNAi molecules is generated. A library of constructs, each comprising an inserted DNA fragment from an insect cell genome, is generated using standard methods. A polymerase chain reaction (PCR) is used to amplify the DNA fragments. The forward and reverse PCR primers contain binding sites for an RNA polymerase, e.g. bacteriophage T7 RNA polymerase. The PCR products are used as templates for the appropriate RNA polymerase, e.g., T7 RNA polymerase, and double-stranded RNA is annealed under conditions that favor complementary RNA/RNA annealing, e.g., 65°C for 30 minutes, followed by slow cooling to room temperature (appr. 22 °C). dsRNA is introduced into cells using standard techniques. One non-limiting example of such a method is as follows. dsRNA is added to cells (at a concentration of about 10⁶ cells/ml) to a final concentration in the range of about 30 to 50 nM, followed by vigorous agitation.

Libraries can be randomly generated and comprise the entire genome, or can comprise a subset of the entire genome. RNAi-based methods of identifying proteins involved in extracytosolic deposition of macromolecules are amenable to high throughput screens. Bargmann (2001) *Genome Biology* 2:1005.1-1005.3.

Where the agent that affects extracytosolic deposition of a macromolecule is an RNAi molecule, identification of the affected protein is carried out by determining the identity of the RNAi molecule, e.g., determining the open reading frame of the RNAi molecule. Since the RNAi molecule is generated from a plasmid construct, the open reading frame of the RNAi molecule is readily identified by determining the open reading frame of the DNA insert in the construct used to generate the RNAi molecule.

As one non-limiting example, S2 cell lines that overexpress HA-tagged chitin synthase are used to screen RNAi libraries for RNAi molecules that affect extracytosolic deposition of the chitin synthase. Example 3 describes an exemplary method for identifying molecules that affect extracytosolic deposition of a macromolecule.

### ACTIVE AGENTS

The invention further provides agents identified using an assay method of the invention. Active agents are useful as pesticides.

Agents of interest are selective for invertebrate pests, e.g., the agent adversely affects an invertebrate pest, yet does not adversely affect a non-target organism, such as a plant or a mammal. An agent that modulates both an invertebrate pest secretory pathway and a mammalian secretory pathway may still selectively adversely affect an invertebrate pest, and thus may be useful as a pesticide if the ADME (absorption, distribution, metabolism, and excretion) is such that the compound adversely affects an invertebrate pest, but not a plant and/or a mammal. Tests for ADME are well known in the art. An agent that is "selective" for an invertebrate pest is an agent that has pharmacologically distinct activities on invertebrate pests (e.g., "target organisms") compared to non-target organisms (e.g., plants and/or mammals) such that the agent adversely affects an invertebrate pest but does not substantially affect viability and/or fertility in a non-target organism.

An agent that "adversely affects" an invertebrate pest adversely affects viability and/or fertility of the invertebrate pest, e.g., viability and/or fertility are reduced by at least about 10%, at least about 20%, at least about 30%, at least about 40%, at least about 50%, at least about 60%, at least about 70%, at least about 80%, or at least about 90% or more when the invertebrate pest is contacted with the agent, compared to the viability and/or fertility of the invertebrate pest when not contacted with the agent. Viability and fertility of an invertebrate pest can be determined using standard assays.

In many embodiments of interest, an agent identified by an assay of the invention is selective for insect pests, i.e., the agent adversely affects an insect pest, but does not substantially adversely affect a non-insect organism.

Agents that prove to be selective for invertebrate pests are formulated for application to an invertebrate pest population. Active agents can be formulated with an acceptable carrier into a pesticidal composition that is, for example, a suspension, a solution, an emulsion, a dusting powder, a dispersible granule, a wettable powder, an emulsifiable concentrate, an aerosol or impregnated granule. Formulations comprising an active agent identified by a screening method of the invention can be applied directly to plants to protect the plants against damage by an invertebrate pest, or can be applied to the soil in which a plant to be protected is grown. Formulations for pesticides are well known in the art, and any known formulation can be used. U.S. Patent No. 6,180,088 describes foamable aerosol formulations for insecticidal compounds.

Such compositions disclosed above may be obtained by the addition of a surface active agent, an inert carrier, a preservative, a humectant, a feeding stimulant, an attractant, an encapsulating agent, a binder, an emulsifier, a dye, a U.V. protectant, a buffer, a flow agent, or other component to facilitate product handling and application for particular target pests.

Suitable surface-active agents include but are not limited to anionic compounds such as a carboxylate, for example, a metal carboxylate of a long chain fatty acid; an N-acylsarcosinate; mono or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate or dioctyl succinate.

Non-ionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitar fatty acid esters, block copolymers of ethylene oxide and propylene oxide, acetylenic glycols such as 2,4,7,9-tetraethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as an amine oxide of polyoxyethylene alkylamine; an amide-linked amine prepared by the condensation of a carboxylic acid with a di- or polyamine; or a quaternary ammonium salt.

Examples of inert materials include but are not limited to inorganic minerals such as kaolin, phyllosilicates, carbonates, sulfates, phosphates or botanical materials such as wood products, cork, powdered corncobs, peanut hulls, rice hulls, and walnut shells.

The compositions of the present invention can be in a suitable form for direct application or as a concentrate or primary powder which requires dilution with a suitable quantity of water or other diluent before application. The insecticidal concentration will vary depending upon the nature of the particular formulation, specifically, whether it is a concentrate or to be used directly. The composition contains 1 to 98% of a solid or liquid inert carrier, and 0 to 50%, preferably 0.1 to 50% of a surfactant. These compositions will be administered at the labeled rate for the commercial product, preferably about 0.01 lb-5.0 lb per acre when in dry form and at about 0.01 pts-10 pts per acre when in liquid form.

The compositions of the invention can be applied directly to the plant by, for example, spraying or dusting at the time when the pest has begun to appear on the plant or before the appearance of pests as a protective measure. Plants to be protected within the scope of the present invention include, but are not limited to, cereals (wheat, barley, rye, oats, rice, sorghum and related crops), beets (sugar beet and fodder beet), drupes, pomes and soft fruit (apples, pears, plums, peaches, almonds, cherries, strawberries, raspberries, and blackberries), leguminous plants (alfalfa, beans, lentils, peas, soybeans), oil plants (rape, mustard, poppy, olives, sunflowers, coconuts, castor oil plants, cocoa beans, groundnuts), cucumber plants (cucumber, marrows, melons), fibre plants (cotton, flax, hemp, jute), citrus fruit (oranges, lemons, grapefruit, mandarins), vegetables (spinach, lettuce, asparagus, cabbages and other brassicae, carrots, onions, tomatoes, potatoes, paprika), lauraceae (avocados, cinnamon, camphor), deciduous trees and conifers (e.g. linden-trees, yew-trees, oak-trees, alders, poplars, birch-trees, firs, larches, pines), or plants such as maize, turf plants, tobacco, nuts, coffee, sugar cane, tea, vines, hops, bananas and natural rubber plants, as well as ornamentals. In some embodiments, a mode of application is by foliar spraying. The mode of application for soil pests can be by furrow application or by "lay-by" application. It is generally important to obtain good control of pests in the early stages of plant growth as this is the time when the plant can be most severely damaged. The spray or dust can conveniently contain another pesticide if this is thought necessary. A pesticidal composition can also be applied directly to the plant.

The compositions of the present invention may be effective against pests including, but not limited to, pests of the order Lepidoptera, e.g. Achroia grisella, Acleris gloverana, Acleris variana, Adoxophyes orana, Agrotis ipsilon, Alabama argillacea, Alsophila pometaria, Amyelois transitella, Anagasta kuehniella, Anarsia lineatella, Anisota senatoria, Antheraea pernyi, Anticarsia gemmatalis, Archips sp., Argyrotaenia sp., Athetis mindara, Bombyx mori, Bucculatrix thurberiella, Cadra cautella, Choristoneura sp., Cochylis hospes, Colias eurytheme, Corcyra cephalonica, Cydia latiferreanus, Cydia pomonella, Datana integerrima, Dendrolimus sibericus, Desmia funeralis, Diaphania hyalinata, Diaphania nitidalis, Diatraea grandiosella, Diatraea saccharalis, Ennomos subsignaria, Eoreuma loftini, Ephestia elutella, Erannis tiliaria, Estigmene acrea, Eulia salubricola, Eupoecilia ambiguella, Euproctis chrysorrhoea, Euxoa messoria, Galleria mellonella, Grapholita molesta, Harrisina americana, Helicoverpa subflexa, Helicoverpa zea, Heliothis virescens, Hemileuca oliviae, Homoeosoma electellum, Hyphantria cunea, Keiferia lycopersicella, Lambdina fiscellaria fiscellaria, Lambdina fiscellaria lugubrosa, Leucoma salicis, Lobesia botrana, Loxostege sticticalis, Lymantria dispar, Macalla thyrsisalis, Malacosoma sp., Mamestra brassicae, Mamestra configurata, Manduca quinquemaculata, Manduca sexta, Maruca testulalis, Melanchra picta, operophtera brumata, Orgyia sp., Ostrinia nubilalis, Paleacrita vernata, Papilio cresphontes, Pectinophora gossypiella, Phryganidia californica, Phyllonorycter blancardella, Pieris napi, Pieris rapae, Plathypena scabra, Platynota flouendana, Platynota sultana, Platyptilia carduidactyla, Plodia interpunctella, Plutella xylostella, Pontia protodiae, Pseudalotia unipuncta, Pseudoplusia includens, Sabulodes aegrotata, Schizura concinna, Sitotroga cerealella, Spilonota ocellana, Spodoptera sp., Thaurnstopoea pityocampa, Tineola bisselliella, Trichoplusia ni, Udea rubigalis, Xylomyges curialis, Yponomeuta padella; Coleoptera, e.g., Leptinotarsa sp., Acanthoscelides obtectus, Callosobruchus chinensis, Epilachna varivestis, pyrrhalta luteola, Cylas formicarius elegantulus, Listronotus oregonensis, Sitophilus sp., Cyclocephala borealis, Cyclocephala immaculata, Macrodactylus subspinosus, Popillia japonica, Rhizotrogus majalis, Alphitobius diaperinus, Palorus ratzeburgi, Tenebrio molitor, Tenebrio obscurus, Tribolium castaneum, Tribolium confusum, Tribolius destructor.

### METHODS OF ISOLATING AN ACTIVE AGENT

The invention further provides methods for isolating and purifying an agent identified by a method of the invention. The methods generally involve identifying an agent using a method as described herein; and purifying the agent.

Any known method of purifying an agent can be used in the subject methods, including, but not limited to, chromatographic methods such as HPLC, gas chromatographic methods, etc.; gel filtration methods; methods based on differential solubility in organic solvents; size exclusion chromatography; and the like.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

### Example 1: Secretion assay

The principle of a secretion assay is outlined in Figure 1. To monitor the transportation and secretion process of proteins in insect cells, a stably transfected Sf-9 cell line is proposed having a secreted Alkaline Phosphatase (AP) under the control of an inducible promoter (e.g. Ecdysone responsive Element). A signal peptide of an insect neuropeptide or an insect secreted peptidase can be used to direct the AP into the secretion pathway. A luciferase enzyme, which is driven by the same promoter in the same cell line, functions as a control to monitor for transcription and translation inhibitors.

### Example 2: Selection of insect specific secretion inhibitors

This assay is depicted schematically in Figure 1.

### Example 3: Chitin synthase assay

The chitin synthase assay is based on the observed effect of an insecticidal compound on the intracellular localization of a Drosophila chitin synthase-hemagglutinin fusion protein (ChS-HA) expressed in Drosophila S2 cells. Specifically, it has been observed that ChS-HA staining shifts from a diffuse/multiple-punctate intracellular distribution to a single intracellular spot following 48 hours of treatment with the insecticidal compound.

The assay uses a stable Hygromycin-selected clone of Drosophila S2 cells that express Drosophila ChS-HA, a recombinant form of ChS that is fused at the C-terminus to a hemagglutinin (HA) tag to allow for immunofluorescent staining of recombinant ChS with an anti-HA antibody. The assay readout is an immunofluorescence endpoint, and requires a fluorescence microscope outfitted with a specialized long working distance 63X objective and fluorescence filters suitable for detecting the fluorescence of the chromophore Cy3.

Plates on which the cells are grown were coated with polyornithine. Compounds (in DMSO) and cells in growth medium (final density of 1 x 10⁶ cells/ml) were added to the coated plates. After 3 days incubation at 22°C, the medium was aspirated, the cells were fixed (4% paraformaldehyde for 15 minutes; 100% methanol for 15 minutes), washed, and blocking agent (5% goat serum) was added. The next day, cells were stained with primary (mouse monoclonal anti-HA) and secondary (Cy3-conjugated goat anti-mouse IgG) antibodies, and analyzed by fluorescence microscopy for the "one-spot" (aggregates) phenotype. The results show that ChS-HA staining shifts from a diffuse/multiple-punctate intracellular distribution to a single intracellular spot following 48 hours of treatment with the insecticidal compound. The shift from a multipunctate arrangement to an aggregate arrangement indicates that export of chitin synthase from the cytosol is disrupted by the compound.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. An *in vitro* assay method for identifying an agent that modulates extracytosolic deposition of a macromolecule in an invertebrate cell, the method comprising:
a) contacting an invertebrate cell with a test agent, which invertebrate cell provides a detectable readout for distribution of the macromolecule in the cell; and
b) determining the effect of the test agent on the distribution of the macromolecule in the cell, wherein an effect on the distribution of the macromolecule, in comparison to a control invertebrate cell not contacted with the agent, indicates that the agent modulates extracytosolic deposition of the macromolecule in an invertebrate cell.

2. The method of claim 1, wherein the cell is selected from the group consisting of Sf-9 cells, Sf-21 cells, S2 cells, Dmel, Kc, Hv1, Hv6, and Hi-5 cells.

3. The method of claim 1, wherein the cell is present in an intact invertebrate organism.,

4. The method of claim 1, wherein said determining step comprises determining an altered subcellular distribution of the macromolecule compared to control cells.

5. The method of claim 4, wherein said macromolecule is detected extracellularly.

6. The method of claim 4, wherein said macromolecule is detected in the cytoplasm of the cell.

7. The method of claim 4, wherein said macromolecule is detected in a subcellular organelle.

8. The method of claim 1, wherein the macromolecule is a protein.

9. The method of claim 8, wherein said altered subcellular distribution of said protein is detected by visual imaging of the protein.

10. The method of claim 9, wherein said protein is detected by binding the protein to a labeled specific binding partner.

11. The method according to claim 10, wherein the specific binding partner is an antibody.

12. The method of claim 1, wherein the altered subcellular distribution is detected using a microscope and observing a single intracellular aggregrate of the protein compared to control cells that exhibit a diffuse, multiple-punctate intracellular distribution of the macromolecule.

13. The method of claim 12, wherein the macromolecule is a protein, and wherein prior to observing the cells using the microscope, the cells are fixed and contacted with an antibody specific to the protein, and wherein said detectable read-out is provided by detecting said antibody binding to the protein.

14. The method of claim 1, wherein the altered subcellular distribution is detected using automated image acquisition hardware.

15. The method of claim 1, wherein said cell comprises a recombinant expression vector comprising a nucleotide sequence that encodes a fusion protein, wherein said fusion protein comprises a detectable marker protein fused in-frame to a secretion signal.

16. The method of claim 1, wherein said cell comprises a recombinant expression vector comprising a nucleotide sequence that encodes a fusion protein, wherein said fusion protein comprises a detectable marker protein fused in-frame to a protein that normally enters a secretory pathway.

17. The method of one of claims 15 or 16, wherein said detectable marker is selected from the group consisting of an immunological tag, a binding site, an enzyme that yields a detectable product, and a fluorescent protein.

18. The method of claim 1, wherein the cell comprises a first recombinant vector comprising a nucleotide sequence that encodes a first fusion protein comprising a signal peptide and a first marker protein, and a second recombinant vector comprising a nucleotide sequence that encodes a second marker protein, wherein an altered subcellular distribution of the first marker protein, and a lack of an effect on levels of the second marker protein, indicates that the test agent specifically modulates a secretory pathway in the cell.

19. The method of claim 18 wherein said first marker protein and said second detectable proteins are each encoded by nucleotide sequences operably linked to an inducible promoter.

20. The method of claim 19, wherein the inducible promoter is selected from the group consisting of a hormone-inducible promoter, a heat-inducible promoter, a metallothionein-inducible promoter, and a tetracycline-inducible promoter.

21. The method of claim 1, further comprising testing the agent in a counter screen using a mammalian cell line to determine whether the test agent affects a extracytosolic deposition in a mammalian cell line.

22. An agent identified using the method according to claim 1.

23. The agent according to claim 22, wherein the agent is a pesticidal agent.

24. An *in vitro* assay method for identifying a modifier protein of an invertebrate secretory pathway, the method comprising:
a) contacting an invertebrate cell that provides a detectable readout for distribution of a marker protein with an agent that specifically inhibits a putative modifier protein; and
b) determining the effect of the agent on the distribution of the marker protein, wherein an effect on the distribution of the marker protein, in comparison to a control cell not contacted with the agent, indicates that the inhibited protein is a modifier of an invertebrate secretory pathway.

25. The method according to claim 20, wherein the agent is selected from the group consisting of a morpholino oligonucleotide, a double-stranded interfering RNA molecule, an antibody, an antisense nucleic acid molecule, and a dominant negative protein domain.
